(19) Europäisches Patentamt
European Patent Office
Office européen des brevets

(11) EP 4 454 546 A1

(12) EUROPEAN PATENT APPLICATION

(43) Date of publication:
**30.10.2024 Bulletin 2024/44**

(21) Application number: **23169438.1**

(22) Date of filing: **24.04.2023**

(51) International Patent Classification (IPC):
***A61B 5/0215*** $^{(2006.01)}$ ***A61B 5/00*** $^{(2006.01)}$

(52) Cooperative Patent Classification (CPC):
**A61B 5/0215; A61B 5/7203; A61B 5/725; A61B 5/7253**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **Koninklijke Philips N.V.**
**5656 AG Eindhoven (NL)**

(72) Inventors:
- **FRANCK, Christoph Florian**
  **Eindhoven (NL)**
- **SZUTTOR, Kai**
  **Eindhoven (NL)**

(74) Representative: **Philips Intellectual Property & Standards**
**High Tech Campus 52**
**5656 AG Eindhoven (NL)**

# (54) ARTEFACT REDUCTION IN INVASIVE BLOOD PRESSURE MEASUREMENT

(57) A signal processing operation for reducing presence of underdamping and catheter whip artefacts in a blood pressure signal obtained from an invasive blood pressure measurement apparatus. A signal transformation for reducing artefacts is configured by fitting parameters of the transformation according to an optimization in which a value of a first function applied to the signal after transformation is minimized, while keeping a value of a second function applied to the signal after transformation to be less than a threshold value. The first function is chosen as a function correlated with a peak value of the signal over a signal window or correlated with a variability of the signal about a trendline over a window, and the second function is chosen as a function correlated with average or aggregate value of the whole signal over a signal window.

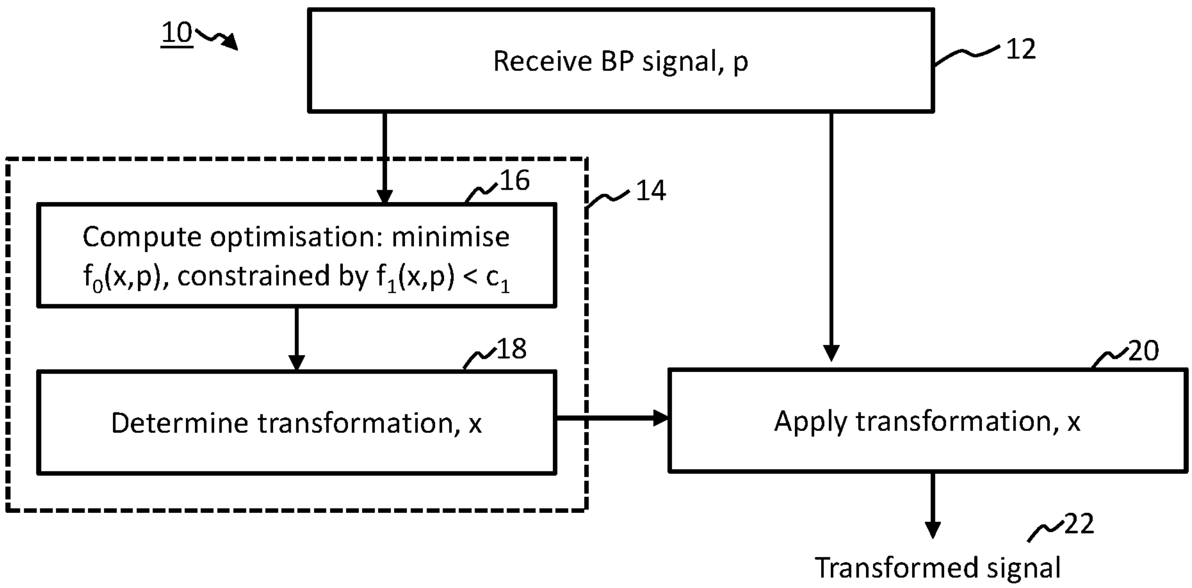


FIG. 1

EP 4 454 546 A1

## Description

FIELD OF THE INVENTION

The present invention relates to the field of invasive blood pressure measurement, and in particular to signal processing within the field of invasive blood pressure measurement.

BACKGROUND OF THE INVENTION

The present invention aims to provide a development in the field of invasive blood pressure measurement. In particular, the claimed method aims to provide the effect of improved accuracy of obtained invasive blood pressure measurements by means of a new method for signal processing aiming at removing or minimizing two common types of signal artefact.

Invasive blood pressure (IBP) refers to a clinical measurement in which a fluid-filled catheter is inserted into an artery of the patient. The catheter is connected to a system of fluid-filled tubing outside the patient's body that acts as a conductor for the intra-arterial pressure. The blood pressure inside the artery is measured by a pressure transducer connected to the tubing system. This measurement is invasive, but is faster, more direct and more accurate than known non-invasive methods for measuring blood pressure.

IBP recorders can measure not only intra-arterial pressure, but also the pressure inside atria and ventricles of the heart, as well as venous pressure.

The IBP measurement is sensitive to the hydraulic behavior of the catheter and tubing system. Excessive tubing length or small air bubbles trapped in the tubing system can change the frequency response of the setup, leading to underdamping. This manifests as the recorded pressure wave overshooting the actual pressure in the blood vessel after sudden pressure changes, such as systole. This may typically therefore manifest as an overshoot in the magnitude of the peak pressure within a particular heart cycle.

The IBP measure is furthermore sensitive to movement of the catheter tip, which causes brief but large oscillations/fluctuations or spike-like artefacts in the recorded pressure wave. These can in some cases have an appearance which is similar to artefacts caused by underdamping, although caused by a different underlying mechanism. Both types of artefacts are well known and described in literature.

Both underdamping and catheter whip artefacts adversely affect the ability to interpret and analyze the pressure waveform, for example determining the systolic, diastolic and mean blood pressure. Especially systolic and mean blood pressure can be significantly overestimated when one or both artefacts are present. Pathological conditions may be masked by the artefacts and necessary treatment may be delayed or erroneously omitted. Conversely, the artefacts may lead to false positives in diagnosis, whereupon a patient may receive unnecessary and potentially harmful treatment.

Currently, underdamping and catheter whip artefacts are addressed by adaptation of the hardware including choosing a catheter and tubing system which is not excessively long, using stiff tubing with an appropriate diameter, and by flushing the tubing system to remove any tapped air bubbles. Hydraulic damping devices are also known which can be inserted into the tubing system to provide additional damping (e.g. the resonance over-shoot eliminator (R.O.S.E), from Medline Industries Inc.).

The document WO 2012/032533 A1 describes one method for automatic detection and correction of underdamping. However, the disclosed method is complex, requiring identification of multiple reference points within the measured pressure curve. This makes the process sensitive to noise or strong artefacts.

A simplified approach to reducing presence of underdamping and/or catheter whip artefacts would be of value.

SUMMARY OF THE INVENTION

The invention is defined by the independent claims. The dependent claims define advantageous embodiments.

According to examples in accordance with an aspect of the invention, there is provided a method for reducing artefacts in an invasive blood pressure measurement, comprising:

receiving a blood pressure signal indicative of a pressure inside a portion of the cardiovascular system of a subject;
performing a signal transformation operation, x, comprising application of a signal transformation to the received blood pressure signal to transform it to a transformed signal, the signal transformation being configurable,
wherein the signal transformation, x, is configured based on performance of an optimization operation comprising:

minimizing, with respect to the transformation x, the value of a first function $f_0$ (x, p) applied to the pressure signal p after application of the signal transformation x, while simultaneously constraining the value of a second function $f_1$ (x, p) applied to the pressure signal p after application of the signal transformation x to be less than a pre-defined threshold value $c_1$,
where $f_0$ is a function which includes at least one component which is correlated with a maximum value of the transformed pressure signal, p, or with a degree of fluctuation about a trend line of the transformed signal over one or more pressure pulses, e.g. over a single pressure pulse, and where $f_1$ is a function which includes at least one component which is correlated with an average or aggregate value of the transformed pressure signal, e.g. over one or more pressure pulses, e.g. over a single pressure pulse.

The inventors have recognized that it is possible to detect the presence of the relevant artefacts by using a simple function which is selected to be sensitive to the artefacts in question, in particular sensitive to variations in peak amplitude of the signal over a single cycle (and thus responsive to overshoot artefacts) and sensitive to variability of the signal about the trendline over a single heart cycle (and thus responsive to catheter whip fluctuations). At the same time, another function can be chosen which is relatively insensitive to the presence of the artefacts, in particular a function which is correlated to average or aggregate signal value. The inventors have recognized that parameters of a signal transformation, such as a filter or a mapping can be fit by running an optimization in which a value of the first function, applied to the transformed signal, is minimized, while constraining a change in the value of the second function, applied to the transformed signal, to less than a threshold amount. This has the effect of seeking to minimize the artefacts while keeping change to the underlying signal content to less than a threshold amount.

Thus, as defined, $f_0$ is chosen as a function which is known to exhibit a (relatively large) increase in value in response to the presence of underdamping or catheter whip artefacts, and $f_1$ is chosen as a function which is known to exhibit relatively less increase in response to the presence of underdamping or catheter whip artefacts (i.e., is largely unaffected by the relevant artefacts).

In some embodiments, the signal transformation operation, x, comprises application of a digital filter, wherein the optimization operation comprises fitting coefficients of the digital filter. The digital filter may be a linear digital filter.

Here, x may be understood as a vector defining the coefficients of the digital filter.

In an alternative approach, the optimization is applied directly to the pressure signal so that the signal transformation may be understood as a sample-to-sample mapping between pressure measurement samples in the received pressure signal and transformed pressure measurement samples in the transformed signal. This achieves an estimation or reconstruction of the pressure signal without the artefacts.

In some embodiments, the first function, $f_0$, is a weighted sum of two or more components, wherein each component is a sub-function which exhibits independently a correlation with a maximum value of the transformed pressure signal, p, or with a degree of fluctuation about a trend line of the transformed signal.

In some embodiments, at least one component of $f_0$ includes a function selected from a first group comprising:

the infinity norm of the transformed pressure signal ($f(x, p) = \|x * p\|_\infty$);
the Lp-norm of the transformed pressure signal for p>=2 ($f(x, p) = \|x * p\|_n$, $n \geq 2$); and
a difference between a maximum and minimum value of the transformed pressure signal

$$\max(x * p) - \min(x * p).$$

The functions of this first group are all functions which are sensitive to overshoot in the peak value of the measured pressure pulse wave, which results in a maximum value of the signal increasing significantly compared to a 'true' signal. Overshoot artefacts such as this are a symptom of underdamping.

The infinity norm represents the largest absolute value of the transformed pressure signal.

With regards to a Lp-norm with p greater than or equal to 2, this function responds strongly to outlier values in the signal, thus picking up overshoots in the signal.

As the skilled person will appreciate, the infinity norm is effectively an edge case of the Lp-norm with p greater than 2.

In some embodiments, at least one component of $f_0$ includes a function selected from a second group comprising: any Lp norm of the first or higher derivative of the transformed pressure signal, and an arc length of the transformed pressure signal. With regards to the Lp norm of the first or higher derivative of the transformed pressure signal, this includes, for example, a total variation of the transformed pressure signal (L1 norm of the first derivative of the transformed pressure signal); a maximum absolute variation of the transformed pressure signal (this is the L_infinity norm of the first derivative of the transformed pressure signal); and includes any Lp norm of the second or higher derivative of the transformed pressure signal. The derivative referred to above may typically be a numeric derivative, since the pressure signal and the transformed pressure signal each consist of a series of discrete sample points.

Total variation means the sum of absolute differences between the pressure samples forming the transformed pressure signal. One way of understanding this is as an indirect way of targeting a total path length of the signal over the relevant time window. In an underdamped wave, this describes a longer path length due to the additional oscillations/fluctuations about the underlying signal trend.

The total variation may be expressed mathematically as the L1 norm of the numeric derivative of a vector of sampled pressure values: $f_1$ **(x, p)** = $\|\Delta(\mathbf{x} * \mathbf{p})\|_1$, after application of the transformation x, where $\Delta\mathbf{p}$ is the numeric derivative, as a function of time, of a vector, **p,** of the sampled pressure values forming the pressure signal, p after application of the transformation, x.

The functions of the second group are unified in all targeting a metric related to a degree of fluctuation or variation of the signal.

In some embodiments, the function $f_0$ comprises a weighted sum of at least one function from the first group and at least one function from the second group.
This way, the resulting function $f_0$ is sensitive to both key types of artefacts which arise in the present context: overshoots and erroneous signal fluctuations. The weightings could be set equal or could be varied relative to one another.

In some embodiments, at least one component of the second function $f_1$ includes an L1 norm of the transformed pressure signal. This is a simple function which essentially indicates a sum of the absolute values of the transformed signal, and thus correlates with an average or aggregate value of the signal. The value of this function is expected to change only relatively little in the presence of artefacts because the differences compared to a signal absent the artefacts would be expected to average out over the whole of a given pulse waveform.

Another aspect of the invention is a processing device.

The processing device comprises an input/output for receiving a blood pressure signal indicative of a pressure inside a portion of a cardiovascular system of a subject. The processing device further comprises a signal transformation module adapted to perform a signal transformation operation, x, comprising application of a signal transformation x to the received blood pressure signal to transform it to a transformed signal, the signal transformation being configurable. The processing device further comprises an optimization module for configuring the signal transformation, x, applied by the signal transformation module, the optimization module adapted to perform an optimization operation comprising: minimizing, with respect to the signal transformation, x, the value of a first function $f_0$ **(x, p)** applied to the pressure signal p after application of the signal transformation, x, while simultaneously constraining the value of a second function $f_1$ (x, p) applied to the pressure signal p after application of the signal transformation, x, to be less than a pre-defined threshold value $c_1$.

The function $f_0$ is a function which includes at least one component which is correlated with a maximum value of the transformed pressure signal, p, or with a degree of fluctuation about a trend line of the signal, e.g., over a single pressure pulse. The function $f_1$ is a function which includes at least one component which is correlated with an average or aggregate value of the transformed pressure signal, e.g., over a single pressure pulse.

In some embodiments, at least one component of $f_0$ includes a function selected from a first group comprising:

the infinity norm of the transformed pressure signal ($f(x, p) = \|x * p\|_\infty$);
the Lp-norm of the transformed pressure signal for p>=2 ($f(x, p) = \|x * p\|_n$, $n \geq 2$); and
a difference between a minimum and maximum value of the transformed pressure signal.

In some embodiments, at least one component of $f_0$ includes a function selected from a second group comprising: any Lp norm of the first or higher derivative of the transformed pressure signal, and an arc length of the transformed pressure signal.

In some embodiments, the function $f_0$ comprises a weighted sum of at least one function from the first group and at least one function from the second group.

In some embodiments, at least one component of $f_1$ includes an L1 norm of the transformed pressure signal.

Another aspect of the invention is a system comprising an invasive blood pressure measurement apparatus; a processing device in accordance with any embodiment described in this document, arranged to receive a blood pressure measurement signal generated by the invasive blood pressure measurement apparatus.

These and other aspects of the invention will be apparent from and elucidated with reference to the embodiment(s) described hereinafter.

## BRIEF DESCRIPTION OF THE DRAWINGS

For a better understanding of the invention, and to show more clearly how it may be carried into effect, reference will now be made, by way of example only, to the accompanying drawings, in which:

Fig. 1 outlines steps of an example method in accordance with one or more embodiments of the invention;
Fig. 2 is a block diagram of an example processing device and system in accordance with one or more embodiments of the invention;

Fig. 3 outlines a set of example blood pressure signals containing signal artefacts; and

Fig. 4 illustrates the effect of application of a signal transformation configured in accordance with a method of one or more embodiments of the invention to each of the pressure signals of Fig. 3.

## DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a signal processing operation for reducing presence of underdamping and catheter whip artefacts in a blood pressure signal obtained from an invasive blood pressure measurement apparatus. A signal transformation for reducing artefacts is configured by fitting parameters of the transformation according to an optimization in which a value of a first function applied to the signal after transformation is minimized, while keeping a value of a second function applied to the signal after transformation to be less than a threshold value. The first function is chosen as a function correlated with a peak value of the signal over a signal window or correlated with a variability of the signal about a trendline over a window, and the second function is chosen as a function correlated with average or aggregate value of the whole signal over a signal window.

An aim of embodiments of the present invention is to provide a means for automatically detecting underdamping and catheter whip artefacts, potentially provide a warning to a user as to the presence of the artefact, and to compensate or reduce the artefacts in the pressure signal. However, at the same time, a method is sought which does not significantly alter signals that are unaffected by these artifacts, i.e. normally damped signals.

According to embodiments of the present invention, it is proposed to automatically generate and apply a signal transformation operator, e.g. by configuring coefficients of a digital filter, so as to correct underdamped signals or signals affected by catheter whip artefacts. The defined optimization operation configures parameters of the transformation by minimizing one function whose value is indicative of the presence of the artefacts while constraining changes in the value of a second function whose value is less affected or unaffected by the presence of the artefacts. Thus, modifying signals without the artefacts is avoided or limited to only insignificant changes. The method can also be used to detect the presence of significant underdamping or catheter whip artefacts and provide a warning to the user of the device.

Embodiments of the invention thus propose to custom-configure an artefact reduction signal transform using a constrained optimization approach in which a first function sensitive to artefacts is minimized and wherein a value of a second function insensitive to artefacts (and thus reflective of the underlying 'true' signal) is constrained. The value of the second function is preferably constrained so as to constrain a difference between the value of the second function applied to the transformed signal and the value of the second function applied to the original untransformed signal.

Embodiments of the invention may improve invasive blood pressure (IBP) signal reliability, for example in patient monitors and other devices with invasive blood pressure measurement functionality. This includes hemodynamic measurement systems which derive parameters such as cardiac output and cardiac stroke volume from IBP wave data.

Fig. 1 outlines in block diagram form steps of an example method according to one or more embodiments. The steps will be recited in summary, before being explained in further detail.

Proposed is a method 10 for reducing artefacts in an invasive blood pressure measurement.

The method 10 comprises receiving 12 an internal blood pressure (BP) signal, p, indicative of a pressure inside a portion of the cardiovascular system.

The method further comprises performing 20 a signal transformation operation, x, comprising application of signal transformation to the received blood pressure signal, p, to transform it to a transformed signal 22. This signal transformation is configurable, for example by varying a set of parameters of the signal transformation. For example, the signal transformation is a mathematical operator with configurable parameters.

The method comprises an optimization or fitting operation 14 in which the signal transformation, x, is configured or determined. For example, parameters of the signal transformation are configured.

The optimization operation may be a constrained optimization procedure.

The optimization operation may comprise minimizing 16, with respect to the transformation x, the value of a first function $f_0$ (x, p) applied to the pressure signal p after application of the signal transformation x, while simultaneously constraining the value of a second function $f_1$ (x, p) applied to the pressure signal p after application of the signal transformation x to be less than a pre-defined threshold value $c_1$. The first function may be understood as a loss function, within the context of a constrained optimization problem. It is noted, by way of completeness, that although minimization is referred to above, a minimization problem can also be converted to an equivalent maximization problem by simply changing the sign of the cost function.

The optimization operation may be run for example iteratively so as to fit parameters for the signal transformation, x, so as to optimize the minimization task for $f_0$ within the defined constraint for $f_1$. The final result determines 18 the transformation function.

The function $f_0$ is a function which includes at least one component which is correlated with a maximum value of the transformed pressure signal, p, or with a degree of fluctuation about a trend line of the signal. The function $f_1$ is a function which includes at least one component which is correlated with an average or aggregate value of the transformed pressure signal, e.g., over a single pressure pulse in the signal or a defined plurality of pressure pulses.

The aforementioned maximum value of the pressure signal may mean a maximum of a peak of a pressure pulse in the transformed pressure signal. The aforementioned degree of fluctuation about a trend line of the signal may mean a degree of fluctuation as determined over a single pulse or a defined plurality of pulses.

The optimization or fitting procedure 14 may in some examples be run once, for example at a start of a measurement session with a patient, and wherein subsequently the same signal transformation is applied throughout the measurement session to received pressure signals. Alternatively, the optimization or fitting procedure 14 may be executed multiple times throughout a measurement session, for example at regular intervals.

As noted above, the method can also be embodied in hardware form, for example in the form of a processing device which is configured to carry out a method in accordance with any example or embodiment described in this document, or in accordance with any claim of this application.

To further aid understanding, Fig. 2 presents a schematic representation of an example processing device 32 configured to execute a method in accordance with one or more embodiments of the invention. The processing device is shown in the context of a system 30 which comprises the processing device. The processing device alone represents an aspect of the invention. The system 30 is another aspect of the invention. The provided system need not comprise all the illustrated hardware components; it may comprise only a subset of them.

The processing device 32 comprises one or more processors 36 configured to perform a method in accordance with that outlined above, or in accordance with any embodiment described in this document or any claim of this application. In the illustrated example, the processing device further comprises an input/output 34 or a communication interface.

In the illustrated example of Fig. 2, the system 30 further comprises an invasive blood pressure measurement apparatus 44 adapted to generate a blood pressure measurement signal 42.

The system 30 may further comprise a memory 38 for storing computer program code (i.e. computer-executable code) which is configured for causing the one or more processors 36 of the processing unit 32 to perform the method as outlined above, or in accordance with any embodiment described in this disclosure, or in accordance with any claim.

In the illustrated example, the one or more processors are shown including a signal transformation module 52 for transforming the pressure signal, **p,** and an optimization module 54 for determining or configuring the signal transformation which is applied by the signal transformation module.

The optimization module 54 is adapted to perform an optimization operation comprising minimizing, with respect to the transformation x, the value of the first function $f_0$ (**x, p)** applied to the pressure signal **p** after application of the signal transformation **x,** while simultaneously constraining the value of the second function $f_1$ (**x**, **p)** applied to the pressure signal p after application of the signal transformation **x** to be less than a pre-defined threshold value $c_1$. The signal transformation module 52 is adapted to perform a signal transformation operation, **x,** comprising application of the configured signal transformation **x** to the received blood pressure signal to transform it to a transformed signal.

As mentioned previously, the invention can also be embodied in software form. Thus another aspect of the invention is a computer program product comprising computer program code configured, when run on a processor, to cause the processor to perform a method in accordance with any example or embodiment of the invention described in this document, or in accordance with any claim of this patent application.

Implementation details in accordance with at least one set of embodiments will now be discussed in more detail.

Embodiments of the invention are based on the observation that it is possible to define mathematical functions of the pressure signal that either strongly change in value as the pressure signal becomes underdamped or becomes subject to catheter whip artefacts (i.e. have high sensitivity to artefacts), or are affected very little (i.e. have low sensitivity to the artefacts).

Such functions are used in an optimization process to estimate the underlying, 'true' pressure signal.

In at least one set of embodiments, this estimation process comprises finding digital filter coefficients that, when applied to the pressure signal, strongly reduce the artefacts while only causing minor alterations in the underlying pressure signal. However, a digital filter is just one possible implementation. In general, any transformation operator can be used, as will be explained later. For example, one alternative approach proposes to define a custom transformation consisting for example of a simple mapping applied directly to the pressure signal, which allows for deriving a transformed signal with artefacts suppressed but which does not yield coefficients for a digital filter. The latter approach has the advantage of enabling handling of nonlinear behavior of the reconstruction, but has the disadvantage of not resulting in digital filter coefficients that can subsequently be saved and applied to the pressure signal as it is being recorded. As a consequence, the latter approach may require running the optimization operation only in retrospect, after the signal has been acquired, rather than allowing for real-time processing. A digital filter approach may allow for signal transformation in real time, once the coefficients of the filter have been determined.

In all cases, values for systolic, diastolic, and mean blood pressure extracted from the (transformed) estimated corrected signal more closely reflect the actual pressure signal without underdamping or catheter whip artefacts.

To better understand embodiments of the invention, Fig. 3 illustrates a set of example blood pressure signals, p, each spanning two heart cycles, each obtained from an invasive blood pressure measurement signal.

Fig. 3 (top left) shows a sample arterial pressure signal with marginal underdamping effects. This signal would be considered acceptable in clinical practice. The marginal underdamping manifests as a slight overshoot in the peak values of the signal maxima in each of the two pulses.

Fig. 3 (top right) shows a sample arterial pressure signal with a large, low-frequency overshoot due to underdamping. This manifests as a larger overshoot in the peak values of the signal maxima in each of the two pulses.

Fig. 3 (bottom left) shows a sample arterial pressure signal with pronounced catheter whip artefact during systole phase. This manifests as a local fluctuation in the signal about the general trend line, as indicated at 62a and 62b.

Fig. 3 (bottom right) shows a sample arterial pressure signal with pronounced underdamping as well as a catheter whip artefact during systole. This manifests as an overshoot in the maximum of each pulse peak and also in the presence of local fluctuation in the signal. The catheter whip fluctuations are indicated at 62a-62d.

With regards to the first function, fo(x,p), different options are possible. As mentioned above, a general aim in selection of this function is to identify a function which exhibits a relatively large increase in value when the pressure signal waveform, p, changes from normally damped to underdamped or begins to exhibit catheter whip artefacts.

As mentioned above, underdamping manifests as an overshoot in the maximum value of the pressure signal within a single heart cycle, i.e. a single pulse. Thus, according to some embodiments, it is proposed to define $f_0(x,p)$ so as to include at least one linear component which is correlated with a maximum value of the transformed pressure signal, p. In this way, the value of $f_0(x,p)$ will increase as the maximum value of the transformed pressure signal peak increases, and thus will increase in relationship with the magnitude of any underdamping in the signal.

It is the further recognition of the inventors that certain simple functions exist which can be expected to correlate with a maximum value of the transformed pressure signal, p. These can be categorized as a first group of functions and include the following:

the infinity norm of the transformed pressure signal ($f(x, p) = \|x * p\|_\infty$);
the Lp-norm of the transformed pressure signal for p>=2 ($f(x, p) = \|x * p\|_n$, $n \geq 2$),
the LogSumExp of the transformed pressure signal;
a difference between a minimum and maximum value of the transformed pressure signal.

In the above, for computing the infinity norm, Lp-norm or LogSumExp, the pressure signal, p, may be represented as a vector, **p,** of sampled pressure values, and wherein the Lp-norm, infinity norm, or LogSumExp is computed using this vector. When computing the infinity norm or Lp-norm, a minimum pressure value may be subtracted from each sampled pressure value, so that each pressure sample in the vector **p** represents a deviation from the minimum pressure value.

By way of background, an Lp-norm (also known as the p norm), is a mathematical concept used in vector spaces to define the "size" or "length" of a vector. It is a generalization of the Euclidean norm (L2 norm) and the Manhattan norm (L1 norm). For a given vector z = (z1, z2, ..., zn) and a positive real number p, the Lp-norm (or p norm) is defined as:

$$\|z\|_p = (|z1|^p + |z2|^p + \dots + |zn|^p)^{\frac{1}{p}}$$

where, |.| denotes the absolute value function.

When p = 1, the L1 norm (Manhattan norm) is obtained, and when p = 2, the L2 norm (Euclidean norm) is obtained. As p approaches infinity, the Lp norm converges to the L-infinity norm (also known as the maximum or Chebyshev norm), which is defined as the maximum absolute value of the vector components:

$$\|z\|_\infty = \max\{|z1|, |z2|, \dots, |zn|\}$$

The infinity norm represents the largest absolute value of the transformed pressure signal. With regards to a Lp-norm with p greater than or equal to 2, this function responds strongly to outlier values in the signal, thus picking up overshoots in the signal. The same is also true of the LogSumExp of the transformed pressure signal. This represents the logarithm of the sum of the exponentials of the signal values.

When applying any of these functions as part of the optimization procedure, it will be recognized that the optimization problem to be solved is to minimize a value of the relevant function applied to the pressure signal or vector after transformation with the signal transformation, i.e. minimize fo(x, **p)**, where **(x, p)** is the result of applying signal transformation x to signal p.

As mentioned above, catheter whip artefacts manifest as local fluctuations in the pressure signal part way along the signal pulse.

Thus, according to some embodiments, it is proposed to define $f_0$(x,p) so as to include at least one component which is correlated with a degree of fluctuation about a trend line of the signal, i.e. correlated with a degree of high frequency noise in the signal.

In this way, the value of $f_0$(x,p) will increase as the degree or amount of local fluctuation in the signal increases, and thus will increase in relationship with the magnitude of any catheter whip artefacts in the signal.

It is the further recognition of the inventors that certain simple functions exist which can be expected to correlate with a degree of local fluctuation of transformed pressure signal, p, across a single heart cycle pulse. These can be categorized as a second group of functions and include the following:

- any Lp norm of the first or higher numeric derivative of the transformed pressure signal;
- Huber loss function applied to the numeric derivative values of the transformed pressure signal. This is a smooth approximation to the L1 norm (absolute value function). When applied to a vector of the numeric derivative values, it penalizes the roughness of the function while being less sensitive to outliers compared to using the L1 norm directly; and
- an arc length of the transformed pressure signal.

With regards to Lp norms of the first or higher numeric derivative of the transformed pressure signal, this includes for example the following functions:

- Total variation of the transformed pressure signal. This may be otherwise expressed as the L1 norm of the numeric derivative of a vector, **p,** of the sampled pressure values forming the pressure signal, p, after application of the transformation, x:

$$f_0(\mathbf{x}, \mathbf{p}) = \|\Delta(\mathbf{x} * \mathbf{p})\|_1$$

  where $\Delta(\mathbf{x} * \mathbf{p})$ is the numeric derivative, as a function of time, of a vector, $(\mathbf{x} * \mathbf{p})$, of the sampled pressure values of pressure signal, **p** after application of the transformation, **x;**
- Maximum absolute variation of the transformed pressure signal. This may be otherwise expressed as the L-infinity norm applied to the differences of neighboring values of the transformed pressure signal. This focusses on identifying the largest absolute variation among all the consecutive pairs of values;
- any Lp norm of the second or higher derivative of the transformed pressure signal.

With regards to the total variation, this is a measure of smoothness of a signal. It is defined as the sum of the absolute differences between adjacent samples in the signal. It will be recognized that this is identical to the L1 norm of the numeric derivative of a vector, **p,** of the sampled pressure values forming the pressure signal, p, after application of the transformation, as defined above.

The total variation of a signal can in general be understood as a measure of the amount or degree of change or deviation in the signal. A signal with a high total variation is less smooth or more erratic than a signal with a low total variation.

Total variation, TV, of a signal, p, may be defined as follows:

$$TV(p) = \sum_i |p(i) - p(i-1)|$$

where p(i) represents the i-th sample in the signal.

With regards to the Maximum Absolute Variation, this may be understood mathematically as follows. Given the vector, **p,** of the sampled pressure values forming the pressure signal, **p** with values $p_i$ for i = 1, 2, ..., n, the numeric derivative values can be computed as the differences between consecutive values, i.e., $\Delta p_i = p_{i+1} - f_i$ for i = 1, 2, ..., n-1.

The maximum absolute variation is then defined as:

$$MAV(p) = \max\{|\Delta p_1|, |\Delta p_2|, \ldots, |\Delta p_{n-1}|\}$$

With regards to the Huber loss function, this can be defined as:

$$H(y,\delta) = \begin{Bmatrix} 0.5 * y^2 & \text{if } |y| \leq \delta \\ \delta * |y| - 0.5 * \delta^2 & \text{if } |y| > \delta \end{Bmatrix}$$

where y is the input value, and δ is a positive parameter that controls the transition between the quadratic (smooth) and linear (absolute value) regions of the function.

To apply the Huber loss function to the vector of numeric derivative values of **p**, the process comprises first computing the numeric derivatives $\Delta p_i = p_{i+1} - p_i$ for i = 1, 2, ..., n-1. Then, applying the Huber loss function to each $\Delta p_i$:

$$H(\Delta p, \delta) = \begin{Bmatrix} 0.5 * (\Delta p)^2 & \text{if } |\Delta p_i| \leq \delta \\ \delta * |\Delta p_i| - 0.5 * \delta^2 & \text{if } |\Delta p_i| > \delta \end{Bmatrix}$$

The total Huber loss for the vector of numeric derivative values is the sum of the Huber losses for all $\Delta p_i$:

$$HuberLoss(p,\delta) = \sum_i H(\Delta p_i, \delta) \text{ for } i = 1, 2, \ldots, n-1$$

Here, *HuberLoss*(*p, δ*) is the Huber loss applied to the numeric derivative values of the signal p with parameter δ.

More generally, with regards to the second group of functions, any norm or (quasi-) convex penalty function of higher derivatives of **p** (after transformation by x) could be used, e.g. total or maximum curvature (second derivative).

In each of the above, in practice, when running the optimization, the relevant functions will be applied to the transformed pressure signal, i.e. the pressure signal or vector p after application of the signal transformation, x.

With regards to the arc length, this typically refers to the length of the curve representing the signal as it is plotted in a time-amplitude space. A signal can be continuous, like an analog signal, or discrete, like a digital signal.

For example, in the case of a signal waveform plotted on a graph with the x-axis representing time and the y-axis representing the amplitude of the signal, the curve connecting the points in this graph represents the signal over time. The arc length of this curve is the cumulative distance between all consecutive points on the curve.

For continuous signals, arc length can be determined using calculus, specifically by integrating the square root of the sum of squares of the time and amplitude derivatives over the desired time interval. For discrete signals, arc length can be calculated by summing the Euclidean distances between consecutive points.

The arc length of a signal can be used to quantify the complexity or variability of the signal, with a longer arc length indicating a more complex or rapidly varying signal.

With regards to the second function, $f_1$, different options are possible. As mentioned above, a general aim in selection of this function is to identify a function which exhibits relatively little change in value when artefacts are introduced to a pressure signal to which it is being applied. In other words, this is a function which is insensitive to changes in the presence or magnitude of underdamping and catheter whip artifacts. In other words, it is a function which exhibits relatively little increase in value when the pressure signal waveform, p, changes from normally damped to underdamped or begins to exhibit catheter whip artefacts.

It is the recognition of the inventors that a suitable function, $f_1(x,p)$ having these characteristics would be a function which is indicative of an overall or average or aggregate value of the signal, so that the function $f_1(x,p)$ changes mostly in relationship with changes in the underlying signal component of the pressure signal, as opposed to changing selectively and disproportionately in response to artefact presence.

Accordingly, it is proposed, in accordance with at least one set of embodiments, that $f_1$ is a function which includes at least one linear component which is correlated with an average or aggregate value of the pressure signal over a single heart cycle.

Suitable functions include for example:

- the L1 norm of the pressure sample vector, **p,** after application of the transformation x: ($f(\mathbf{x}, \mathbf{p}) = \|(\mathbf{x} * \mathbf{p})\|_1$); and

- the L1 norm of the difference between the pressure sample vector, **p,** after application of the transformation, **x,** and a vector, $\mathbf{p_0}$, representing the sample values of the original untransformed pressure signal, i.e. a normally damped version of the signal. ($f(\mathbf{x}, \mathbf{p}) = \|(\mathbf{x} * \mathbf{p}) - \mathbf{p}_0\|_1$)

The former results in a non-convex optimization problem, while the latter can be used to set up a convex optimization problem. See: Boyd J, Vandenberghe L. "Convex Optimization", Cambridge University Press 2004.

For both $f_0(x,p)$ and $f_1(x,p)$, it may be preferable to normalize the pressure signal, e.g. the pressure sample vector, **p,** in advance of applying the function. This can be done in particular by subtracting a mean value of the pressure vector from all samples, and then dividing all samples by the standard deviation of the pressure vector values. This makes subsequent calculations scale-invariant and invariant to offsets.

For each of $f_0$ and $f_1$ it is to be noted that the function may optionally comprise a weighted sum of two or more components, wherein each component is a sub-function which exhibits independently a correlation with a maximum value of the transformed pressure signal, p, or with a degree of fluctuation about a trend line of the transformed signal.

For example, $f_0$ may comprise a weighted sum of at least one function from the first group mentioned above and at least one function from the second group mentioned above. This way, the resulting function $f_0$ is sensitive to both key types of artefact which arise in the present context: overshooting and erroneous oscillations. The weightings could be set equal or could be varied relative to one another.

It is noted that the various functions, f, outlined above are all characterized in that they can be expressed as a quasiconvex or quasiconcave function g of a linear or affine mapping of the vector of pressure samples **p:** $f(\mathbf{p}) = g((\mathbf{A} \cdot \mathbf{p}) + \mathbf{b})$.

The set of quasiconcave and quasiconvex functions contains, by the definition of quasiconcavity and quasiconvexity, all concave, convex, affine, and linear functions. The function g need only by quasiconvex or quasiconcave within the conceivable value range of its argument. The mathematical condition of (quasi) convexity/concavity confers advantages to an optimization problem, such as the lack of local minima when the aim is to minimize a (quasi-) convex function. It may be advantageous for the optimization problem itself to be a convex optimization problem, i.e., the cost function and the inequality constraints are convex functions, and any equality constraints are affine ("linear") functions, but this is not essential.

As discussed above, it is proposed to use the functions $f_0$ and $f_1$ as part of a fitting or optimization process for configuring or determining a signal transformation operation, x, which is to be applied to the pressure signal, p, for reducing artefacts in the signal. In particular, it is proposed to minimize, with respect to variation in the transformation **x** (i.e. variation in parameters of the transformation x), the value of a first function $f_0(\mathbf{x}, \mathbf{p})$ applied to the pressure signal p after application of the signal transformation **x,** while simultaneously constraining the value of a second function $f_1(\mathbf{x}, \mathbf{p})$ applied to the pressure signal p after application of the signal transformation x to be less than a pre-defined threshold value $c_1$:

$$\text{minimize } f_0(\mathbf{x}, \mathbf{p}) \qquad \text{w.r.t. } \mathbf{x}$$

$$f_1(\mathbf{x}, \mathbf{p}) \leq c_1$$

where (**x**, **p**) represents the result of application of the transformation x to the pressure signal p.

The value $c_1$ may be defined relative to the value of $f_1$ applied to the original, untransformed version of the pressure signal, p. In other words, $c_1$ may be defined so as to constrain a change in the value of $f_1$ applied to the transformed signal (x,p) as compared to the value of $f_1$ applied to the original untransformed signal, p.

In operation, the method comprises receiving a digitized arterial blood pressure signal, comprising a series of pressure samples. From the received signal may be extracted at least one signal section or portion, each signal section or portion spanning a certain time frame, e.g. 4-8 seconds. Each signal section or portion may span in some examples a certain discrete number of heart cycle pulses, e.g. one or two or three.

The optimization operation may then be performed using at least one extracted signal section or portion. In this document, reference to 'the pressure signal' may be understood as reference to a pressure signal section or portion, for example spanning one or more pulses, each representative of a respective heart cycle.

The optimization problem is to minimize the value of the first function $f_0(x, p)$ with the constraint that the change in value of the second function $f_1$ is small (</= c 1).

The optimization variables are parameters of the signal transformation operator, x, where the signal transformation is for example a parametrized mathematical operator for application to a pressure signal, p, where the pressure signal, p, may be represented in vector form, **p.**

By way of example, the signal transformation operation, x, may be a digital filter, for example a finite impulse response (FIR) filter, for example a time-invariant (LTI) digital finite impulse response (FIR) filter. In this case, the optimization variables are the coefficients of the digital filter. The coefficients of the filter may be represented as a vector x of the filter coefficients.

As mentioned above, for the purposes of the optimization procedure for configuring the signal transformation, the pressure signal vector, **p,** may be normalized in advance of applying the functions, fo, $f_1$. This can be done in particular by subtracting a mean value of the pressure vector from all samples, and then dividing all samples by the standard deviation of the pressure vector values. This makes subsequent calculations scale-invariant and invariant to offsets.

Once the optimization process concludes, the resulting filter coefficients may be applied to the original pressure data to produce the corrected waveform with underdamping or catheter whip artefacts being substantially attenuated.

This optimization problem can be solved using known algorithms such as, for example, active-set methods, interior-point methods, sequential quadratic programming, gradient methods. However, the optimization is not confined to any particular method or class of algorithm.

In general, solving the optimization problems comprises constructing a cost function and seeking to minimize the cost function by perturbing or fitting the optimization variables. In the present case the optimization variables are the configurable parameters of the signal transformation operator, for example the coefficients of a digital filter.

Suitable algorithms for performing the optimization include for instance gradient descent, conjugate gradient, or interior-point methods.

By way of one illustrative example, the following represents example steps of an optimization operation for determining parameters of a transformation, x.

First, the transformation coefficients are initialized with starting values. Theses may be chosen randomly or may be chosen based on prior knowledge of the system.

A search space for the parameters of the signal transformation, x, may be defined. This may be a continuous or a discrete search space depending upon the nature of the transformation.

Next, the value of the objective function, fo, and constraint function, $f_1$ are computed for the initialized values. In particular, for the given solution(s) in the optimization algorithm, the signal transformation x is applied to the pressure signal p, and the values of $f_0(x,\cdot p)$ and fi(x,-p) are calculated. A check is performed as to whether the constraint fi(x,·p) $\leq c_1$ is satisfied.

Next, the current solution(s) are updated using the chosen optimization algorithm. This may involve updating the parameters of the signal transformation x based on the gradient information or other algorithm-specific rules.

The above two steps are iterated until a stopping criterion is met. This could be a maximum number of iterations, a threshold on the improvement in the objective function value, or a convergence criterion.

Once the stopping criterion is met, the best solution found during the optimization process is extracted. This solution should correspond to the optimal parameters of the signal transformation x that minimizes $f_0(x,\cdot p)$ while satisfying the constraint fi(x,·p) $\leq c_1$.

The signal transformation x with the optimized parameters is then applied to the pressure signal p, and obtain the transformed signal.

Optionally, the solution may then be validated by verifying the performance of the optimized signal transformation by comparing the transformed signal to a desired output or performance metric.

The above process represents one example for configuring the parameters of a signal transformation x, to minimize the value of the function $f_0(x,\cdot p)$, while constraining the value of the function $f_1(x,\cdot p)$ to be less than the pre-defined threshold value $c_1$.

With regards the possible optimization algorithms, many possible algorithms are available in the form of ready-to-use libraries. The book "Convex Optimization" gives an introduction to at least some classes of algorithm.

Thus, to summarize, the procedure performed may be as follows. First, an invasive blood pressure signal is received comprising a sequence of pressure samples which may be represented in the form of a pressure sample vector, **p.** The above-discussed optimization problem is set up and solved so as to minimize fo($\mathbf{x} * \mathbf{p}$) with regard to transformation parameters **x** (e.g. filter coefficients), with the constraint that the change of $f_1(\mathbf{x} * \mathbf{p})$ is less than a defined constant, $c_1$. Optionally, where the transformation being configured is a digital filter, a further constraint may be applied that sum(**x**) = 1, where x represents a vector of the filter coefficient. This constrains the gain of the digital filter **x** at DC to 1. The calculated filter coefficients **x** are applied to the pressure wave samples **p.** The resulting filtered wave may be presented to the user via a user interface. The filtered pressure wave may be used to derive hemodynamic parameters such as systolic arterial pressure, diastolic arterial pressure, and mean arterial pressure.

Fig. 4 illustrates a set of example invasive blood pressure measurement signals, p, each spanning two heart cycles, and wherein each illustrated graph shows an original version of the signal (dashed line) overlaid with a transformed/corrected version of the signal (solid line). The signals in each of the illustrated graphs correspond to those shown in Fig. 3.

Fig. 4 (top left) shows a sample arterial pressure signal with marginal underdamping effects before and after processing with a signal transformation in accordance with an embodiment of the invention. Due to the marginal nature of the original artefact, the corrected version of the signal is only marginally changed. However, it can be observed that, in the transformed version of the signal (solid line), the marginal overshoot in the pulse maxima is corrected.

Fig. 4 (top right) shows a sample arterial pressure signal with a large, low-frequency overshoot in the pulse maxima due to underdamping. It can be seen that in the transformed version of the signal (solid line), this overshoot is corrected.

Fig. 4 (bottom left) shows a sample arterial pressure signal with a pronounced catheter whip artefact during systole phase. This manifests, in the original version of the signal (dashed line), as a local fluctuation in the signal about the general trend line, as indicated at 62a and 62b. It can be seen that in the transformed version of the signal (solid line), the fluctuation is substantially smoothed. In addition, an overshoot in the pulse peak is corrected.

Fig. 4 (bottom right) shows a sample arterial pressure signal with pronounced underdamping as well as a catheter whip artefact during systole. This manifests, in the original version of the signal (dashed line), as an overshoot in the maximum of each pulse peak and also in the presence of local fluctuation in the signal. The catheter whip fluctuations are indicated at 62a-62d. It can be seen that in the transformed version of the signal (solid line), the fluctuation is substantially smoothed. In addition, an overshoot in the pulse peak is corrected.

In some embodiments, the above-described optimization procedure may be modified by adding a regularization term to the cost function, fo(x, p).

The regularization term is a function only of the optimization variables (e.g. the parameters of the signal transformation, e.g. filter coefficients). It is therefore distinguished from cost function components that are also functions of the pressure data. The regularization term may for example penalize the L2 norm of the filter coefficients (which corresponds to an average gain of the filter in the frequency domain) or the L2 norm of the difference between the filter coefficient vector and a vector of initial coefficient values. Regularization prevents the optimization algorithm from arriving at extreme, pathological or undesired solutions.

Additionally or alternatively, in some embodiments, constraints may be placed on the optimization variables. For example, in the case in which the signal transformation is a digital filter, so that the optimization variables are the coefficients of a digital FIR filter, bounds constraints may be used to constrain the filter coefficient values to a given value range. This may be used for example to ensure that the coefficient values do not exceed a value range of a data type used for storing the coefficients and applying them to a signal.

In some embodiments, the optimization operation 14 may be performed at regular intervals throughout operation. The behavior of the catheter and tubing system may change over time, and thus by repeating the optimization at intervals, the signal transformation can be updated to provide consistent suppression of artefacts.

However, at the same time, each incidence of updating the signal transformation operation, e.g. switching the filter coefficients, carries a risk of introducing artefacts in a currently measured pressure wave. Thus, it may be advantageous to include functionality for performing a check, before implementing an updated signal transformation, whether an update in the signal transformation operation is necessary. This may comprise, after running the optimization operation at each of the regular intervals for determining proposed new parameters for the transformation, assessing one or more pre-defined update criteria, and only applying the updated signal transformation for subsequent use if the update criteria are met.

The update criteria may include a magnitude of the difference between the current and the new transformation parameters (e.g. the filter coefficients), and the effects of the current and the new transformation parameters on hemodynamic parameter values derived from the pressure wave, e.g. systolic pressure, diastolic pressure and mean arterial pressure. If the new filter coefficients effect no significant changes in the derived hemodynamic parameter values, switching to the new coefficients is not necessary, and vice versa. If the one or more update criteria are met, the method may comprise controlling a smooth switching procedure for transitioning the parameters of the transformation operation from their current values to the newly computed values (e.g., by crossfading or interpolating between current and new filter coefficients) to minimize switching artefacts.

As mentioned above, in some embodiments, the method may comprise generating feedback to a user indicative of the presence of artefacts, for example via control of a user interface. For example, in some embodiments, a user interface may be controlled to present a warning to a clinical user of the system in the event that artefacts are detected as present, and have been corrected by the application of the signal transformation operation. In some embodiments, the feedback may only be generated in the event that detected artefacts exceed a defined magnitude threshold.

In some embodiments, the generated feedback may include guidance information for a user for guiding the user in an action which may correct the physical cause of the artefacts, for example guidance to check the tubing system of the invasive blood pressure measurement apparatus 44, for example for performing flushing of the tubing to remove any air bubbles, to check a positioning of a catheter tip of a measurement catheter comprised by the blood pressure measurement apparatus.

By way of example, in the case that the signal transformation operation comprises application of a digital filter, the method may comprise determining a frequency response of the filter coefficients determined by the optimization operation and generate feedback for the user in the event that the resultant frequency response is not in compliance with applicable standards for pressure measurement.

By way of further example, the method may comprise comparing systolic/diastolic blood pressure values derived from processing of an uncorrected version of the pressure waveform with systolic/diastolic blood pressure values derived from equivalent processing of the corrected pressure waveform (after application of the signal transformation operation) and generating a warning via a user interface if the difference exceeds a pre-defined threshold.

By way of further example, the method may comprise comparing a value of the function $f_1(p)$ applied to the pressure waveform before correction, and a value of the function $f_1(p)$ applied to the pressure waveform after application of the signal transformation operation. If the difference exceeds a predefined threshold, a user alert may be generated via a user interface.

Embodiments discussed above consider a procedure in which a pressure signal section is extracted from a received pressure signal, comprising a continuous sequence of pressure measurement samples over a certain time window (e.g. spanning one or two or three pulse waves). This signal section is then processed by the optimization procedure to determine the transformation operation. According to a further set of embodiments, the configuration of the transformation operation, e.g. of the filter coefficients, may be performed in a sample-by-sample basis, instead of the block-wise approach described previously. This avoids the latency caused by block-wise processing.

To compute the functions $f_0$ and $f_1$ on a sample-by-sample basis, it is possible to adapt the optimization procedure to an online or adaptive processing method. For example, one such method is the Least Mean Squares (LMS) algorithm, which can be used to update the transformation parameters iteratively for each incoming sample.

In general terms, a sample-by-sample approach involves keeping a buffer of previous samples in memory and using these and the most recent sample to update the filter coefficients. Due to the resulting frequent updates, each update step may be computationally simpler than in a block-wise approach. For example, a sample-by-sample algorithm may only perform one iteration step per new sample, while a block-wise algorithm might perform as many iteration steps as necessary to find the optimal point within a given tolerance.

For example, the sequence of steps performed in a sample-wise approach could be roughly summarized by the following pseudo-code:

*Set current solution to initial value*

*Repeat:*

*Receive new sample*

*Append new sample to buffer of previous samples, remove oldest sample from buffer*

*Update solution candidate using current solution and buffer of samples*

*Set current solution to solution candidate*

By contrast, the block-wise approach in pseudo code might be summarized as::

*Receive new block of samples*

*Set current solution to initial value (or to solution of previous block)*

*Repeat:*

*Update solution candidate using current solution and block of samples*

*Set current solution to solution candidate*

*Until current solution satisfies solution tolerance criteria*

With regards to the signal transformation operation, x, an example implementation discussed above is the use of a digital filter, and wherein the optimization procedure comprises configuring the coefficients of the digital filter. However, other implementations of the signal transformation operation may also be considered.

One simple approach may be based on estimating a corrected pressure waveform directly, bypassing use of a digital filter altogether. In practice, this could be achieved by performing an optimization problem in which the optimization variables are the values of the corrected pressure signal instead of the coefficients of a digital filter. In this case, the transformation operation is effectively a custom-defined sample-to-sample mapping, wherein each sample value in the original pressure signal is mapped by the transformation to a modified value in a corrected pressure signal, and wherein the parameters of the transformation operation consist of these sample-to-sample mappings. Thus, the optimization variables in this case are the sample-to-sample mappings, wherein there is one optimization variable per sample in the pressure signal, p.

In this case, the optimization procedure may utilize the L1 norm for the constraining function $f_1$, i.e., to constrain or penalize the L1 norm of a difference between the corrected and the original waveform. The optimization setup may further be configured to constrain or penalize other functions of the corrected and the original waveform, such as the numeric derivative of the corrected pressure wave. This may enable avoidance of large steps in the estimated corrected waveform.

Embodiments of the invention are applicable generally in the field of invasive blood pressure measurement and monitoring, including for example both stationary and mobile general purpose patient monitors, specialized hemodynamic monitoring systems, and defibrillators with integrated IBP monitoring functionality.

Embodiments of the invention described above employ a processing device. The processing device may in general comprise a single processor or a plurality of processors. It may be located in a single containing device, structure or unit, or it may be distributed between a plurality of different devices, structures or units. Reference therefore to the processing device being adapted or configured to perform a particular step or task may correspond to that step or task being performed by any one or more of a plurality of processing components, either alone or in combination. The skilled person will understand how such a distributed processing device can be implemented. The processing device includes a communication module or input/output for receiving data and outputting data to further components.

The one or more processors of the processing device can be implemented in numerous ways, with software and/or hardware, to perform the various functions required. A processor typically employs one or more microprocessors that may be programmed using software (e.g., microcode) to perform the required functions. The processor may be implemented as a combination of dedicated hardware to perform some functions and one or more programmed microprocessors and associated circuitry to perform other functions.

Examples of circuitry that may be employed in various embodiments of the present disclosure include, but are not limited to, conventional microprocessors, application specific integrated circuits (ASICs), and field-programmable gate arrays (FPGAs).

In various implementations, the processor may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

A single processor or other unit may fulfill the functions of several items recited in the claims.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to".

Any reference signs in the claims should not be construed as limiting the scope.

## Claims

1. A method (10) for reducing artefacts in an invasive blood pressure measurement, comprising:

   receiving (12) a blood pressure signal, p, indicative of a pressure inside a portion of the cardiovascular system of a subject, including one or more pressure pulses;
   performing a signal transformation operation (20), x, comprising application of a signal transformation to the received blood pressure signal to transform it to a transformed signal, the signal transformation being configurable,
   wherein the signal transformation, x, is configured (18) based on performance of an optimization operation (14) comprising:
   minimizing (14), with respect to the transformation x, the value of a first function $f_0$ (x, p) applied to the pressure signal p after application of the signal transformation x, while simultaneously constraining the value of a second function $f_1$ (x, p) applied to the pressure signal p after application of the signal transformation x to be less than a pre-defined threshold value $c_1$,
   where $f_0$ is a function which includes at least one component which is correlated with a maximum value of the transformed pressure signal, p, or with a degree of fluctuation about a trend line of the signal over one or more pressure pulses, and where $f_1$ is a function which includes at least one component which is correlated with an average or aggregate value of the transformed pressure signal.

2. The method of claim 1, wherein the signal transformation operation, x, comprises application of a digital filter, wherein the optimization operation comprises fitting coefficients of the digital filter.

3. The method of claim 1 or 2, wherein the first function, fo, is a weighted sum of two or more components, wherein each component is a sub-function which exhibits independently a correlation with a maximum value of the transformed pressure signal, p, or with a degree of fluctuation about a trend line of the transformed signal over a single heart cycle.

4. The method of any of claims 1-3, wherein at least one component of $f_0$ includes a function selected from a first group comprising:

   the infinity norm of the transformed pressure signal ($f(\mathbf{x} * \mathbf{p}) = \|\mathbf{x} * \mathbf{p}\|_\infty$);
   the Lp-norm of the transformed pressure signal for p>=2 ($f(\mathbf{x} * \mathbf{p}) = \|\mathbf{x} * \mathbf{p}\|_n$, n >_ 2); and
   a difference between a minimum and maximum value of the transformed pressure signal.

5. The method of any of claims 1-4, wherein at least one component of $f_0$ includes a function selected from a second group comprising:

   any Lp norm of the first or higher derivative of the transformed pressure signal; and
   an arc length of the transformed pressure signal.

6. The method of claim 4 and claim 5, wherein the function $f_0$ comprises a weighted sum of at least one function from the first group and at least one function from the second group.

7. The method of any of claims 1-6, wherein at least one component of $f_1$ includes an L1 norm of the transformed pressure signal.

8. A processing device (32), comprising:

   an input/output (34) for receiving a blood pressure signal indicative of a pressure inside a portion of a cardiovascular system of a subject, and including one or more pressure pulses;
   a signal transformation module (52) adapted to perform a signal transformation operation, x, comprising application of a signal transformation x to the received blood pressure signal to transform it to a transformed signal, the signal transformation being configurable; and
   an optimization module (54) for configuring the signal transformation, x, applied by the signal transformation module, the optimization module adapted to perform an optimization operation comprising:

minimizing, with respect to the signal transformation, x, the value of a first function $f_0$ (**x**, **p)** applied to the pressure signal p after application of the signal transformation, x, while simultaneously constraining the value of a second function $f_1$ (**x**, **p)** applied to the pressure signal p after application of the signal transformation, x, to be less than a pre-defined threshold value $c_1$,
wherein $f_0$ is a function which includes at least one component which is correlated with a maximum value of the transformed pressure signal, p, or with a degree of fluctuation about a trend line of the signal over one or more pressure pulses, and where $f_1$ is a function which includes at least one component which is correlated with an average or aggregate value of the transformed pressure signal.

**9.** The device of claim 8, wherein at least one component of $f_0$ includes a function selected from a first group comprising:

the infinity norm of the transformed pressure signal ($f(\mathbf{x}, \mathbf{p}) = \|\mathbf{x} * \mathbf{p}\|_\infty$);
the Lp-norm of the transformed pressure signal for p>=2 ($f(\mathbf{x}, \mathbf{p}) = \|\mathbf{x} * \mathbf{p}\|_n$, $n \geq 2$); and
a difference between a minimum and maximum value of the transformed pressure signal.

**10.** The device of claim 8 or 9, wherein at least one component of $f_0$ includes a function selected from a second group comprising:

any Lp norm of the first or higher derivative of the transformed pressure signal; and
an arc length of the transformed pressure signal.

**11.** The device of claim 9 and claim 10, wherein the function $f_0$ comprises a weighted sum of at least one function from the first group and one function from the second group.

**12.** The device of any of claims 8-11, wherein at least one component of $f_1$ includes an L1 norm of the transformed pressure signal.

**13.** A system (30) comprising:

an invasive blood pressure measurement apparatus (44); and
the processing device (32) as claimed of any of claims 8-12, arranged to receive a blood pressure measurement signal (42) generated by the invasive blood pressure measurement apparatus.

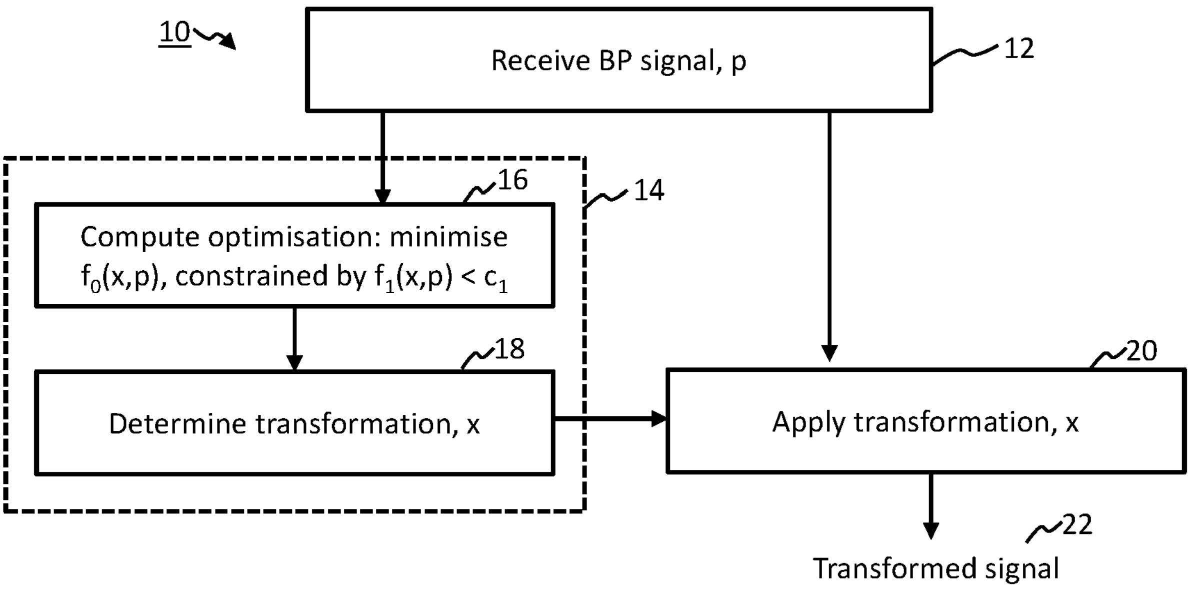
10
Receive BP signal, p
12
16
14
Compute optimisation: minimise $f_0(x,p)$, constrained by $f_1(x,p) < c_1$
18
20
Determine transformation, x
Apply transformation, x
22
Transformed signal

FIG. 1

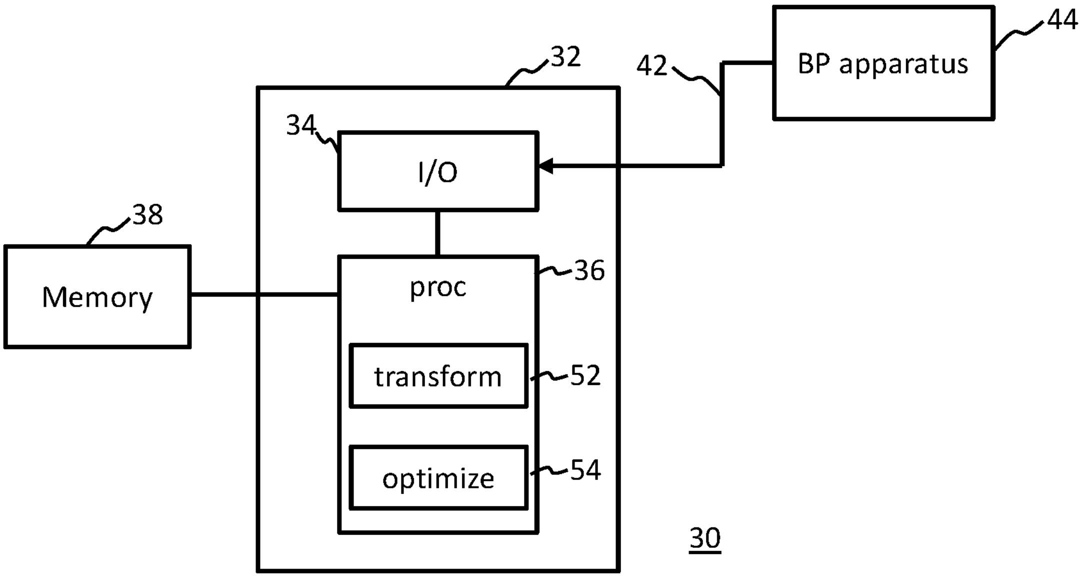
44
32
42
BP apparatus
34
I/O
38
36
Memory
proc
transform
52
optimize
54
30

FIG. 2

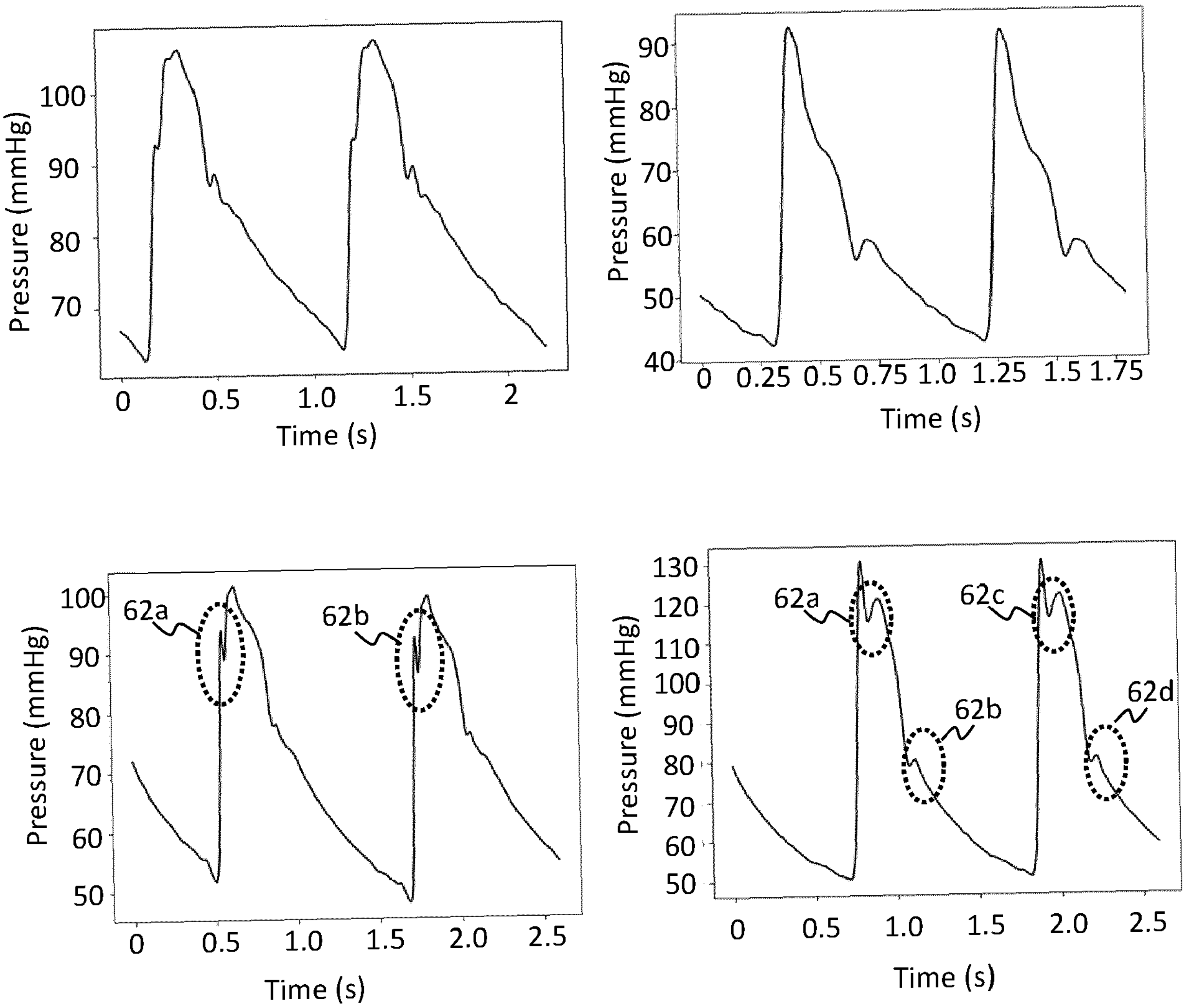
Pressure (mmHg)
100
90
80
70
0
0.5
1.0
1.5
2
Time (s)
Pressure (mmHg)
90
80
70
60
50
40
0
0.25
0.5
0.75
1.0
1.25
1.5
1.75
Time (s)
Pressure (mmHg)
100
90
80
70
60
50
62a
62b
0
0.5
1.0
1.5
2.0
2.5
Time (s)
Pressure (mmHg)
130
120
110
100
90
80
70
60
50
62a
62c
62b
62d
0
0.5
1.0
1.5
2.0
2.5
Time (s)

FIG. 3

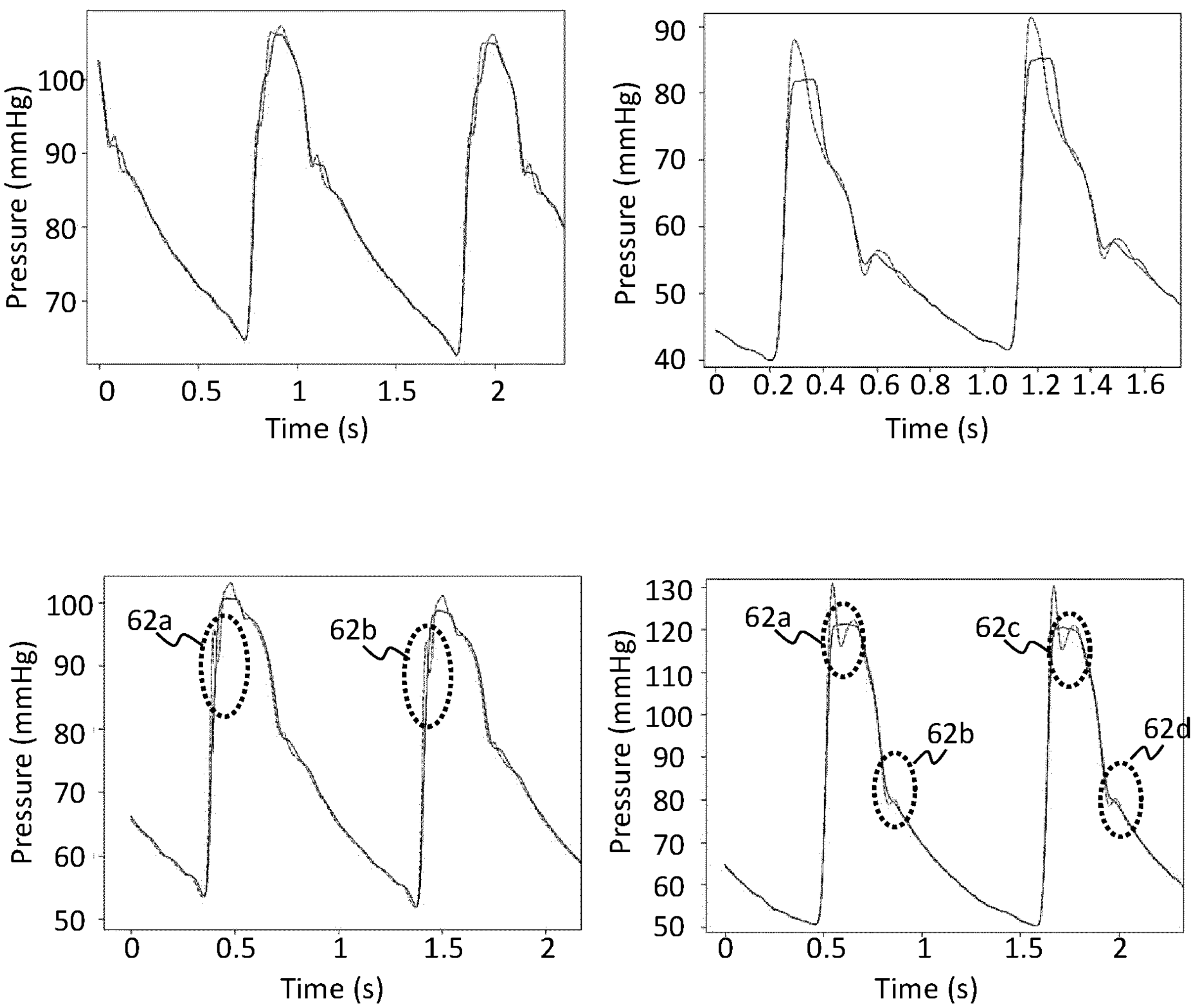
Pressure (mmHg)
100
90
80
70
0
0.5
1
1.5
2
Time (s)
Pressure (mmHg)
90
80
70
60
50
40
0
0.2
0.4
0.6
0.8
1.0
1.2
1.4
1.6
Time (s)
Pressure (mmHg)
100
90
80
70
60
50
62a
62b
0
0.5
1
1.5
2
Time (s)
Pressure (mmHg)
130
120
110
100
90
80
70
60
50
62a
62b
62c
62d
0
0.5
1
1.5
2
Time (s)

FIG. 4

Europäisches Patentamt
European Patent Office
Office européen des brevets

# EUROPEAN SEARCH REPORT

Application Number
EP 23 16 9438

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A,D | WO 2012/032553 A1 (ROMANO SALVATORE [IT]) 15 March 2012 (2012-03-15) * the whole document * ----- | 1-13 | INV. A61B5/0215 A61B5/00 |
| A | US 2022/338742 A1 (JOHNSON DANIEL [US] ET AL) 27 October 2022 (2022-10-27) * abstract * ----- | 1-13 | |

TECHNICAL FIELDS SEARCHED (IPC)

A61B

The present search report has been drawn up for all claims

1

| Place of search | Date of completion of the search | Examiner |
|---|---|---|
| The Hague | 16 October 2023 | Dhervé, Gwenaëlle |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO.

EP 23 16 9438

This annex lists the patent family members relating to the patent documents cited in the above-mentioned European search report. The members are as contained in the European Patent Office EDP file on
The European Patent Office is in no way liable for these particulars which are merely given for the purpose of information.

16-10-2023

| Patent document cited in search report | Publication date | Patent family member(s) | Publication date |
|---|---|---|---|
| WO 2012032553 A1 | 15-03-2012 | AU 2011300338 A1 | 14-03-2013 |
| | | BR 112013005065 A2 | 07-06-2016 |
| | | BR 112013005200 A2 | 03-05-2016 |
| | | CA 2809930 A1 | 15-03-2012 |
| | | CN 103108585 A | 15-05-2013 |
| | | CN 103200864 A | 10-07-2013 |
| | | EP 2613690 A1 | 17-07-2013 |
| | | EP 2613691 A1 | 17-07-2013 |
| | | ES 2503572 T3 | 07-10-2014 |
| | | ES 2578998 T3 | 03-08-2016 |
| | | HK 1187225 A1 | 04-04-2014 |
| | | IT 1402427 B1 | 04-09-2013 |
| | | PL 2613690 T3 | 31-03-2015 |
| | | PL 2613691 T3 | 30-11-2016 |
| | | RU 2013115110 A | 20-10-2014 |
| | | RU 2013115364 A | 20-10-2014 |
| | | US 2013150736 A1 | 13-06-2013 |
| | | US 2013172761 A1 | 04-07-2013 |
| | | US 2017086685 A1 | 30-03-2017 |
| | | WO 2012032386 A1 | 15-03-2012 |
| | | WO 2012032553 A1 | 15-03-2012 |
| US 2022338742 A1 | 27-10-2022 | US 2022338742 A1 | 27-10-2022 |
| | | WO 2021062086 A1 | 01-04-2021 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2012032533 A1


### Non-patent literature cited in the description


- **BOYD J ; VANDENBERGHE L.** Convex Optimization. Cambridge University Press, 2004